# EUROPEAN PATENT SPECIFICATION

(11) **EP 0 420 600 B1**
(45) Date of publication and mention of the grant of the patent: **16.04.1997**
(21) Application number: 90310516.1
(22) Date of filing: 26.09.1990
(51) Int. Cl.: A61K 38/55

(54) **Use of protease inhibitors as antiexudative, antiphlogistic and antimicrobial agents**
Verwendung von Protease-Inhibitoren als antiexudative, antiphlogistische und antimikrobielle Mittel
Utilisation des inhibiteurs des protéases comme agents anti-exudatifs, antiphlogistiques et antimicrobiens

(30) Priority: 29.09.1989 CS 5552/89
(43) Date of publication of application: 03.04.1991
(73) Proprietor: Academy of Sciences of the Czech Republic, 111 42 Praha Stare Mesto (CZ)
(72) Inventor: Cejkova, Jitka, c/o Czechosl. Academy of Sciences, CS-11142 Praha Stare Mesto (CS); Vacik, Jiri, c/o Czechosl. Academy of Sciences, CS-11142 Praha Stare Mesto (CS); Lojda, Zdenek, c/o Czechosl. Academy of Sciences, CS-11142 Praha Stare Mesto (CS)
(74) Representative: Overbury, Richard Douglas

(56) References cited:
- EP-A- 0 223 254
- AMERICAN JOURNAL OF OTOLARYNGOLOGY, vol. 9, no. 3, 1988, pages 142-148; W.B. Saunders Comp.; Y. HAMAGUCHI et al.: "Antiinflammatory effects of a corticosteroid and protease inhibitor agents on antigen-induced otitis media in Chinchillas"
- JOURNAL OF PHARMACOBIO-DYNAMICS, vol. 5, no. 5, May 1982, Pharmaceutical Soc. of Japan, pages 319-327; H. NAKAGAWA et al.: "Effect of proteinase inhibitors having anti-inflammatory activity on gelatinase, elastase and cathepsin G isolated from rat polymorphonuclear leukocytes"
- BIOCHEMICAL PHARMACOLOGY, vol. 32, no. 1, 1st April 1983, Pergamon Press, pages 1191-1195; H. NAKAGAWA et al.: "Anti-inflammatory effect of proteinase inhibitors on carrageenin-induced inflammation in rats"

## Description

The invention pertains to a pharmaceutically active composition and more particularly to a medicamentous form of the composition for external use and having strong antiexudation, antiphlogistic, and antimicrobial effects. The composition is provided in a suitable aqueous or ointment base and is applicable, for example, as an ophthalmologic, otolaryngologic, or dermatologic drug.

Arachidic acid is liberated in damaged, wounded or inflamed tissues from phospholipids of cytoplasmatic membranes by the action of phospholipase enzyme, and may be then metabolized by the cyclooxygenase cycle (by cyclooxygenase enzyme) or the lipooxygenase cycle (by lipooxygenase enzyme) to prostanoids and eicosanoids.

Antiphlogistics of both the steroid and nonsteroid nature, antibiotics, and sulfonamides are often used for therapeutic purposes. The antibiotics, which specifically suppress pathogenic microbes and which are most often used in ophthalmology, include tetracycline, chloramphenicol, bacitracin, and neomycin. Therapeutic substances which prevent the development of inflammation (antiphlogistics) are known in both steroidal and nonsteroidal forms. The steroidal antiphlogistics (for example dexamethasone) block in particular phospholipase. The antiinflammatory drugs of nonsteroid nature (e.g., indomethacin, flurbiprofen, pirprofen) block in particular cyclooxygenase. The blockade of these enzymes is important, because the products formed in metabolic cycles have a strong chemotactic effect (they cause accumulation of leucocytes (e.g., some leucotrienes) in the sites of origin) and increase the vascular permeability. This contributes to an excessive development of inflammation. Inflammations (both of infectious and noninfectious origin) are very dangerous for the anterior and posterior segments of the eye. Thus, scars formed in the cornea as the final stage of healing processes cause the loss of an exceptional function of this tissue, i.e. of its transparency. The loss of transparency of optical media of the eye (cornea, lens) then leads to a reduction in or even loss of sight.

Disadvantages of locally applied conventional antiphlogistics are their relatively low efficiency, retardation of healing, and their contribution to the development of infection. The local effect of antibiotics has been found to be limited and, moreover, there is a danger of the development of an allergic reaction. A higher concentration of antibiotics, which is necessary for obtaining the healing effect in many cases, can have toxic effects on the tissue. For this reason, a local treatment with antibiotics is commonly supplemented by a general administration of antibiotics, which has disastrous consequences with respect to the suppression of antibody formation and the ability of the organism to combat infection. This is why new methods of treatment are desirable. One of the possibilities for use in the treatment of inflammation is the inhibition of plasmin and other destruction proteases (e.g., collagenase or elastase) with specific inhibitors. The said enzymes either directly develop the destruction processes (e.g., plasmin) or enable or activate these processes by their own activity (e.g., collagenase, elastase). However, plasmin is effective not only as an initiator of the development of the chain of degeneration processes, but also contributes to an excessive development of inflammation by several other mechanisms, of which at least chemotaxis should be mentioned.

Amongst others, plasmin may be inhibited with aprotinin. This substance when administered in an aqueous solution and low concentrations has been successfully used in the treatment of some lesions of the anterior segment of eye. See EP-A-0223254 which discloses the use of 5 to 200 IU/ml (0.0007 to 0.028 mg/ml) aprotinin for such use. The authors of this invention found, moreover, that aprotinin inhibits not only plasmin but also several other proteolytic enzymes (for example, leucocytic elastase), which contribute to the destruction processes.

Accordingly, the present invention seeks to provide a pharmaceutically active composition which can be put into a medicamentous form, in an aqueous or ointment base, particularly suitable as an ophthalmologic, otolaryngologic, and dermatologic drug, which contains, inhibitors of proteases.

According to the present invention there is provided a pharmaceutically active composition suitable for use in an ophthalmologic, otolaryngologic or dermatologic medicament having anti-exudation, antiphlogistic and/or anti-microbial properties, which composition comprises an inhibitor of proteases in physiological saline or buffer solution or in an ointment base characterised in that the inhibitor of proteases is selected from elastatinal, elastatinal and aprotinin, and elastatinal and soya bean trypsin inhibitor and is present in a concentration of 0.1 to 20mg of inhibitor per 1ml of physiological saline or buffer solution or per 1g of ointment base.

There is also provided a medicament containing the composition of the present invention.

The inhibitors may be applied either singly or in combination, dissolved in physiological saline or buffer solution with pH 6.5 to 7.5, which is advantageously ionically balanced (e.g., phosphate or borax buffer) or present in an ointment base.

The ionically balanced buffer solution means that sodium chloride is added to the buffer solution in such a way, that the resulting solution is ionically balanced, for example, a precise formulation for a borax buffer with pH 7.4 is as follows:
solution
- A -: 1.9 g Na₂P₄O₇ per 100 ml H₂O pro injectione
- B -: 1.25 g H₃BO₃ + 0.3 g NaCl per 100 ml H₂O pro injectione
- 10 ml of solution A + 90 ml of solution B.

The composition and its medicamentous form according to the invention in liquid state may further advantageously contain 0.05 to 15 wt.-% of thickeners selected from the group comprising hydroxypropyl methyl cellulose, methyl cellulose, polyvinylpyrrolidone, polyvinyl alcohol, poly(alklene glycols), poly/hydroxyalkyl(meth)acrylates or poly(meth)acrylamides.

High concentrations of elastatinal alone, or also with the further specified inhibitors, locally applied can act not only curably with respect to the advanced stage of disease but also preventively, i.e. they can prevent the formation or onset of tissue destruction processes if administered in time. The vehicles (thickeners) with protracted effect then enable a longer contact of the remedy (e.g. aprotinin) with the tissue.

The combination of elastatinal with other inhibitors, e.g. with aprotinin or inhibitor from soya-beans, enhances the therapeutic effect.

The pharmaceutically active composition according to the invention may advantageously further comprise 0.05 to 1.5 wt.-% of steroidal antiphlogistics, for example, indomethacin, and/or 0.2 to 1 wt.-% of antibiotics killing microbes, for example, bacitracin, neomycin, tetracycline, or chloramphenicol.

The combination of the protease inhibitor elastatinal alone or with the other specified inhibitors with antiphlogistics or antibiotics or all the substances together increases the anti-inflammatory and anti-microbial effect (the inhibitors block some products of microbes, e.g. elastase and other proteases). This permits the use of antibiotics only locally and in smaller doses. The concentration of antiphlogistics may also be reduced and, at the same time, the therapeutic effect is greater and the time of treatment may be shorter, which is of particular importance for the healing of tissue.

The composition or its medicamentous form is most often applied by instillation or as an ointment into the conjunctival sac. However, it can be also used for irrigation or lubrication of the eye, facial sinuses, and external auditory meatus, and it may be injected into the anterior eye chamber, and the like. The composition or its medicamentous form in the liquid state may also be presented in the form of a hydrophilic three-dimensional polymer matrix, advantageously in the form of a strip, contact lens, and the like, from which the active components are released in use. The incorporation of the composition or its medicamentous form into a hydrophilic matrix may be performed according to the invention, for example, by conditioning of the matrix in a solution of the composition or its medicamentous form in order to attain the required concentration of inhibitors, or also antiphlogistics and antibiotics, in the polymer matrix.

Pharmaceutically active composition according to the invention have a strong antiexudative, antiphlogistic and antimicrobial effect. Their therapeutic effects consist above all in the inhibition of plasmin, leucolytic elastase, and other serine proteases, and in the inhibited activation of latent forms of some endoproteases and several further subsequent reactions such as chemotaxis and vasculatisation of the cornea. The pharmaceutically active composition according to the present invention prevents in many cases the development of some diseases and, in other cases, stops the development of disease.

The invention is further illustrated in the following exemplary examples, without being limited in its scope to them.

### Preparation of medicamentous form in liquid state:

Each substance is separately dissolved in a small amount (10 to 40 ml) of buffer or physiological saline.

### Ointment base and ointment preparation:

10 g lanolin, 10 g liquid paraffin, and 80 g white petroleum jelly is melted in a water bath, the mixture is strained through hydrophilic gauze and then sterilized. If the therapeutic ingredient is easily soluble in water, it is dissolved in the necessary amount of distilled water for the preparation of injections, mixed with the ointment base in part molten form in a water bath and stirred until complete cooling is effected. If the therapeutic ingredient is insoluble in water, it is used for this preparation in its finest powdered form and first triturated in a smaller amount of liquid paraffin and then mixed with the ointment base.

### Example 1

Aprotinin 0.025 g
elastatinal 0.005 g
hydroxypropyl methyl cellulose 2.5 g
ionically balanced borax buffer up to 100 g
The drops applied into the conjunctival sac every 4 hours were successfully used for the treatment of corneal infiltrates which disappeared during a week.

### Example 2

Aprotinin 0.004 g
inhibitor from soya beans 0.1 g
hydroxypropyl methyl cellulose 1.5 g
elastatinal 0.01 g
ionically balanced phosphate buffer up to 100 g
The drops were used for the treatment of a cornea etched with concentrated acids and hydroxides. The cornea healed during a month and the transparency was recovered either completely or in part.

### Example 3

Aprotinin 0.1 g
elastatinal 0.01 g
prednisolone acetate 0.02 g
polyvinylpyrrolidone (mol. weight 360,000) 1 g
ionically balanced phosphate buffer of pH 7.4 up to 100 g
The drops were instilled 4 times a day for the treatment of deep corneal infiltrates. Healing occurred within a week. In cases complicated with a secondary inflammation of the iris, also this inflammation disappeared during a week.

### Example 4

Aprotinin 1 g
dexamethasone sodium phosphate 0.1 g
indomethacin 1 g
elastatinal 0.1 g
A contact lens comprising HEMA - DEGMA (the crosslinked copolymer of 2-hydroxyethyl methacrylate with diethylene glycol methacrylate containing 55 wt.-% of equilibrium water) was applied to an eye etched with strong alkalies and the drops were instilled over a contact lens at intervals of 4 h. The lesion healed during 3 weeks.

### Example 5

Aprotinin 1 g
tetracycline 0.3 g
elastatinal 0.2 g
flurbiprofen 0.5 g
hydroxypropyl methyl cellulose 3 g
ionically balanced phosphate buffer (pH 7.4) up to 100 g
The drops were instilled at intervals of 3 h into eyes etched with concentrated hydroxides. The cornea did not break down and healed with a scar during a month.

### Example 6

Elastatinal 0.5 g
dexamethasone sodium phosphate 0.1 g
polyvinylalcohol 2 g
physiological saline up to 100 g
The drops were instilled into the conjunctival sac at intervals of 3 h. Perforation of the cornea healed during a week.

### Example 7

Elastatinal 0.3 g
soya-bean inhibitor of trypsin 0.2 g
hydroxypropyl methyl cellulose 2 g
ionically balanced phosphate buffer (pH 7.4) up to 100 g
The drops were instilled into the conjunctival sac 4 times a day. Defects of the corneal epithelium healed during a week.

### Example 8

Aprotinin 0.1 g
elastatinal 0.05 g
dexamethasone sodium phosphate 0.1 g
chloramphenical 0.5 g
physiological saline up to 100 g
The solution was successfully used in the treatment of rhinal allergoses and allergoses of meatus acusticus externus.

### Examples 9

Elastatinal 0.1 g
aprotinin 0.1 g
dexamethasone sulfate 0.15 g
flurbiprofen 0.1 g
chloramphenicol
physiological saline up to 100 g
A contact lense made from the crosslinked copolymer of 2-hydroxyethyl methacrylate and diethylene glycol methacrylate (HEMA - DEGMA) (55 wt.-% of equilibrium water content) was swelled in this solution for 24 h. The contact lens was used for the treatment of corneae burned with alkalis and lime. The healing ad integram occurred in some cases; in more severe cases, where the cornea usually had broken down, the tissue after the application of lenses healed with a scar. In other cases, the contact lens proved very suitable for the treatment of corneal ulcers.

### Example 10

Aprotinin 1 g
elastatinal 0.1 g
flurbiprofen 0.1 g
dexamethasone sodium phosphate 0.1 g
neomycin sulphate
physiological saline up to 100 g
A contact lens from the crosslinked polymer of 2-hydroxyethyl methacrylate (38 wt.-% of equilibrium water) was swelled in the given solution for 24 h. The contact lens was used for the treatment of non-healing corneal erosions. Within a week after application, the reepithalization was accelerated. Extra-ordinary results were attained after severe etching and burning of the anterior segment of eye. An intraocular inflammation did not develop, the cornea did not exhibit ulceration and healed with a scar during a month. The transparency recovered in the periphery of corneae.

### Example 11

Aprotinin 0.02 g
elastatinal 0.05 g
dexamethasone sulphate 0.1 g
chloramphenicol 0.2 g
ionically balanced phosphate buffer (pH 7.4) up to 100 g
A strip from HEMA - DEGMA material (55 wt.-% of equilibrium water content) was immersed for 24 h into the solution of the above given composition and then applied into the lower fornix of the eye. After 24 h the strip was removed from eye, immersed into the given solution overnight, and applied again. Corneal ulcers of various origin were healed after a week of treatment (application).

## Claims (Claims for the following Contracting State(s): AT, BE, CH, DE, DK, FR, GB, IT, LI, LU, NL, SE)

1. A pharmaceutically active composition suitable for use in an ophthalmologic, otolaryngologic or dermatologic medicament having anti-exudation, antiphlogistic and/or anti-microbial properties, which composition comprises an inhibitor of proteases in physiological saline or buffer solution or in an ointment base characterised in that the inhibitor of proteases is selected from elastatinal, elastatinal and aprotinin, and elastatinal and soya bean trypsin inhibitor and is present in a concentration of 0.1 to 20mg of inhibitor per 1ml of physiological saline or buffer solution or per 1g of ointment base.

2. A composition according to claim 1, wherein the buffer solution is ionically balanced and has a pH in the range of from 6.5 to 7.5.

3. A composition according to any preceding claim, wherein the composition further comprises from 0.05 to 1.5 wt% of a steroidal antiphlogistic agent.

4. A composition according to claim 3, wherein the steroidal antiphlogistic agent is dexamethasone.

5. A composition according to any preceding claim, wherein the composition further comprises from 0.05 to 5 wt% of a non-steroidal antiphlogistic agent.

6. A composition according to claim 5, wherein the non-steroidal antiphlogistic agent is indomethacin.

7. A composition according to any preceding claim, wherein the composition further comprises from 0.2 to 1 wt% of an antibiotic agent.

8. A composition according to claim 7, wherein the antibiotic agent is neomycin, bacitracin, chloramphenicol or tetracycline.

9. A composition according to any preceding claim, wherein the composition further comprises from 0.05 to 15 wt% of thickeners selected from hydroxypropyl methyl cellulose, methyl cellulose, polyvinylpyrrolidone, polyvinylalcohol, poly(alkylene glycols), poly[alkylene glycol (meth)acrylates] and poly(meth)acrylamides.

10. A medicament comprising a composition according to any preceding claim.

11. A composition or medicament according to any preceding claim when present in a hydrophillic matrix, which matrix is advantageously in the form of a strip or contact lens.

12. The use of a composition or medicament according to any preceding claim, for the manufacture of a medicament for antiexudation, antiphlogistic and/or antimicrobial ophthalmologic, otolaryngologic or dermatologic treatment.

## Claims (Claims for the following Contracting State(s): ES, GR)

1. A method of preparing a pharmaceutically active composition suitable for use in an ophthalmologic, otolaryngologic or dermatologic medicament having antiexudation, antiphlogistic and/or antimicrobal properties, which method comprises admixing at least one inhibitor of proteases and physiological saline or buffer solution or an ointment base characterised in that the inhibitor of proteases is selected from elastatinal, elastatinal and aprotinin, and elastatinal and soya bean trypsin inhibitor and is admixed in an amount of 0.1 to 20mg of inhibitor per 1ml of physiological saline or buffer solution or per 1g of ointment base.

2. A method according to claim 1, wherein the buffer solution is ionically balanced and has a pH in the range of from 6.5 to 7.5.

3. A method according to claim 1 or 2, which further comprises admixing in the composition from 0.05 to 1.5 wt% of a steroidal antiphlogistic agent.

4. A method according to claim 3, wherein the steroidal antiphlogistic agent is dexamethasone.

5. A method according to any preceding claim, which further comprises admixing in the composition from 0.05 to 5 wt% of a non-steroidal antiphlogistic agent.

6. A method according to claim 5, wherein the non-steroidal antiphlogistic agent is indomethacin.

7. A method according to any preceding claim, which further comprises admixing in the composition from 0.2 to 1 wt% of an antibiotic agent.

8. A method according to claim 7, wherein the antibiotic agent is neomycin, bacitracin, chloramphenicol or tetracycline.

9. A method according to any preceding claim, which further comprises admixing in the composition from 0.05 to 15 wt% of at least one thickener selected from hydroxypropyl methyl cellulose, methyl cellulose, polyvinylpyrollidone, polyvinylalcohol, poly(alkylene glycols), poly[alkylene glycol(meth)acrylates] and poly(meth)acrylamides.

10. A method according to any preceding claim, which further comprises forming the composition into a medicament.

11. A method according to any preceding claim, which further comprises impregnating the composition or medicament into a hydrophilic matrix, which matrix is advantageously in the form of a strip or contact lens.

12. The use of a composition or medicament as formed by any preceding claim for the manufacture of a medicament for antiexudation, antiphlogistic and/or antimicrobial ophthalmologic, otolaryngologic or dermatologic treatment.

## Patentansprüche (Patentansprüche für folgende(n) Vertragsstaat(en): AT, BE, CH, DE, DK, FR, GB, IT, LI, LU, NL, SE)

1. Pharmazeutisch wirksame Zusammensetzung, die sich zur Verwendung in ophthalmologischen, otolaryngologischen oder dermatologischen Arzneimitteln mit antiexudativen, antiphlogistischen und/oder antimikrobiellen Eigenschaften eignet, wobei die Zusammensetzung einen Proteaseninhibitor in physiologischer Kochsalz- oder Pufferlösung oder in einer Salbengrundlage enthält, dadurch **gekennzeichnet**,
daß der Proteaseninhibitor unter Elastatinal, Elastatinal und Aprotinin und Elastatinal und Sojabohnen-Trypsininhibitor ausgewählt ist und in einer Konzentration von 0,1 bis 20 mg Inhibitor pro 1 ml physiologische Kochsalz- oder Pufferlösung oder pro 1 g Salbengrundlage vorliegt.

2. Zusammensetzung nach Anspruch 1, wobei die Pufferlösung ionisch ausgeglichen ist und einen pH-Wert im Bereich von 6,5 bis 7,5 hat.

3. Zusammensetzung nach einem der vorhergehenden Ansprüche, wobei die Zusammensetzung ferner 0,05 bis 1,5 Gew.-% eines steroidischen antiphlogistischen Mittels enthält.

4. Zusammensetzung nach Anspruch 3, wobei das steroidische antiphlogistische Mittel Dexamethason ist.

5. Zusammensetzung nach einem der vorhergehenden Ansprüche, wobei die Zusammensetzung ferner 0,05 bis 5 Gew.-% eines nichtsteroidischen antiphlogistischen Mittels enthält.

6. Zusammensetzung nach Anspruch 5, wobei das nichtsteroidische antiphlogistische Mittel Indomethacin ist.

7. Zusammensetzung nach einem der vorhergehenden Ansprüche, wobei die Zusammensetzung ferner 0,2 bis 1 Gew.-% eines antibiotischen Mittels enthält.

8. Zusammensetzung nach Anspruch 7, wobei das antibiotische Mittel Neomycin, Bacitracin, Chloramphenicol oder Tetracyclin ist.

9. Zusammensetzung nach einem der vorhergehenden Ansprüche, wobei die Zusammensetzung ferner 0,05 bis 15 Gew.-% Verdicker enthält, die unter Hydroxypropylmethylcellulose, Methylcellulose, Polyvinylpyrrolidon, Polyvinylalkohol, Poly(alkylenglykolen), Poly[alkylenglykol(meth)acrylaten] und Poly(meth)acrylamiden ausgewählt sind.

10. Arzneimittel, das eine Zusammensetzung nach einem der vorhergehenden Ansprüche enthält.

11. Zusammensetzung oder Arzneimittel nach einem der vorhergehenden Ansprüche, sofern in einer hydrophilen Matrix vorhanden, wobei die Matrix vorteilhafterweise in Form von Streifen oder Kontaktlinsen vorliegt.

12. Verwendung einer Zusammensetzung oder eines Arzneimittels nach einem der vorhergehenden Ansprüche zur Herstellung eines Arzneimittels zur antiexudativen, antiphlogistischen und/oder antimikrobiellen ophthalmologischen, otolaryngologischen oder dermatologischen Behandlung.

## Patentansprüche (Patentansprüche für folgende(n) Vertragsstaat(en): ES, GR)

1. Verfahren zur Herstellung einer pharmazeutisch wirksamen Zusammensetzung, die sich zur Verwendung in ophthalmologischen, otolaryngologischen oder dermatologischen Arzneimitteln mit antiexudativen, antiphlogistischen und/oder antimikrobiellen Eigenschaften eignet, bei dem mindestens ein Proteaseninhibitor und physiologische Kochsalz- oder Pufferlösung oder eine Salbengrundlage vermischt werden,
dadurch **gekennzeichnet**,
daß der Proteaseninhibitor unter Elastatinal, Elastatinal und Aprotinin und Elastatinal und Sojabohnen-Trypsininhibitor ausgewählt ist und in einer Konzentration von 0,1 bis 20 mg Inhibitor pro 1 ml physiologische Kochsalz- oder Pufferlösung oder pro 1 g Salbengrundlage vermischt wird.

2. Verfahren nach Anspruch 1, wobei die Pufferlösung ionisch ausgeglichen ist und einen pH-Wert im Bereich von 6,5 bis 7,5 hat.

3. Verfahren nach Anspruch 1 oder 2, bei dem ferner in die Zusammensetzung 0,05 bis 1,5 Gew.-% eines steroidischen antiphlogistischen Mittels eingemischt werden.

4. Verfahren nach Anspruch 3, wobei das steroidische antiphlogistische Mittel Dexamethason ist.

5. Verfahren nach einem der vorhergehenden Ansprüche, bei dem ferner in die Zusammensetzung 0,05 bis 5 Gew.-% eines nichtsteroidischen antiphlogistischen Mittels eingemischt werden.

6. Verfahren nach Anspruch 5, wobei das nichtsteroidische antiphlogistische Mittel Indomethacin ist.

7. Verfahren nach einem der vorhergehenden Ansprüche, bei dem ferner in die Zusammensetzung 0,2 bis 1 Gew.-% eines antibiotischen Mittels eingemischt werden.

8. Verfahren nach Anspruch 7, wobei das antibiotische Mittel Neomycin, Bacitracin, Chloramphenicol oder Tetracyclin ist.

9. Verfahren nach einem der vorhergehenden Ansprüche, bei dem ferner in die Zusammensetzung 0,05 bis 15 Gew.-% mindestens eines Verdickers eingemischt werden, der/die unter Hydroxypropylmethylcellulose, Methylcellulose, Polyvinylpyrrolidon, Polyvinylalkohol, Poly(alkylenglykolen), Poly[alkylenglykol(meth)acrylaten] und Poly(meth)acrylamiden ausgewählt ist/sind.

10. Verfahren nach einem der vorhergehenden Ansprüche, bei dem ferner aus der Zusammensetzung ein Arzneimittel hergestellt wird.

11. Verfahren nach einem der vorhergehenden Ansprüche, bei dem ferner eine hydrophile Matrix mit der Zusammensetzung oder dem Arzneimittel imprägniert wird, wobei die Matrix vorteilhafterweise in Form von Streifen oder Kontaktlinsen vorliegt.

12. Verwendung einer nach einem der vorhergehenden Ansprüche hergestellten Zusammensetzung oder Arzneimittels zur Herstellung eines Arzneimittels zur antiexudativen, antiphlogistischen und/oder antimikrobiellen ophthalmologischen, otolaryngologischen oder dermatologischen Behandlung.

## Revendications (Revendications pour l'(les) Etat(s) contractant(s) suivant(s): AT, BE, CH, DE, DK, FR, GB, IT, LI, LU, NL, SE)

1. Composition pharmaceutiquement active appropriée à une utilisation dans un médicament ophtalmologique, otolaryngologique ou dermatologique ayant des propriétés d'anti-exsudation, antiphlogistiques et/ou antimicrobiennes, ladite composition comprenant un inhibiteur de protéases dans un sérum physiologique ou dans une solution tamponnée ou dans une base d'onguent, caractérisée en ce que l'inhibiteur de protéases est choisi parmi l'élastatinal, l'élastatinal et l'aprotinine, et l'élastatinal et un inhibiteur de trypsine provenant de graines de soja, et est présent à une concentration de 0,1 à 20 mg d'inhibiteur par ml de sérum physiologique ou de solution tamponnée ou par gramme de base d'onguent.

2. Composition selon la revendication 1, dans laquelle la solution tamponnée est ioniquement équilibrée et a un pH compris entre 6,5 et 7,5.

3. Composition selon l'une quelconque des revendications précédentes, comprenant, en outre, de 0,05 à 1,5 % en poids d'un agent antiphlogistique stéroïdien.

4. Composition selon la revendication 3, dans laquelle l'agent antiphlogistique stéroïdien est la dexaméthasone.

5. Composition selon l'une quelconque des revendications précédentes, comprenant, en outre, de 0,05 à 5 % en poids d'un agent antiphlogistique non-stéroïdien.

6. Composition selon la revendication 5, dans laquelle l'agent antiphlogistique non-stéroïdien est l'indométacine.

7. Composition selon l'une quelconque des revendications précédentes, comprenant, en outre, de 0,2 à 1 % en poids d'un agent antibiotique.

8. Composition selon la revendication 7, dans laquelle l'agent antibiotique est la néomycine, la bacitracine, le chloramphénicol ou la tétracycline.

9. Composition selon l'une quelconque des revendications précédentes, comprenant, en outre, de 0,05 à 15 % en poids d'épaississants choisis parmi l'hydroxypropylméthylcellulose, la méthylcellulose, la polyvinylpyrrolidone, l'alcool polyvinylique, les poly(alkylèneglycols), les poly[(méth)acrylates d'alkylèneglycol] et les poly(méth)acrylamides.

10. Médicament comprenant une composition selon l'une quelconque des revendications précédentes.

11. Composition ou médicament selon l'une quelconque des revendications précédentes, qui est présent dans une matrice hydrophile ayant avantageusement la forme d'une bande ou d'une lentille de contact.

12. Utilisation d'une composition ou d'un médicament selon l'une quelconque des revendications précédentes pour la fabrication d'un médicament pour un traitement anti-exsudatif, antiphlogistique et/ou antimicrobien en ophtalmologie, en otolaryngologie ou en dermatologie.

## Revendications (Revendications pour l'(les) Etat(s) contractant(s) suivant(s): ES, GR)

1. Procédé de préparation d'une composition pharmaceutiquement active appropriée à une utilisation dans un médicament ophtalmologique, otolaryngologique ou dermatologique ayant des propriétés d'anti-exsudation, antiphlogistiques et/ou antimicrobiennes, comprenant le mélange d'au moins un inhibiteur de protéases et d'un sérum physiologique ou d'une solution tamponnée ou d'une base d'onguent, caractérisé en ce que l'inhibiteur de protéases est choisi parmi l'élastatinal, l'élastatinal et l'aprotinine, et l'élastatinal et un inhibiteur de trypsine provenant des graines de soja, et est ajouté en une quantité de 0,1 à 20 mg d'inhibiteur pour 1 ml de sérum physiologique ou de solution tamponnée ou pour 1 g de base d'onguent.

2. Procédé selon la revendication 1, dans lequel la solution tamponnée est ioniquement équilibrée et a un pH compris entre 6,5 et 7,5.

3. Procédé selon la revendication 1 ou 2, comprenant, en outre, l'incorporation, dans la composition, de 0,05 à 1,5 % en poids d'un agent antiphlogistique stéroïdien.

4. Procédé selon la revendication 3, dans lequel l'agent antiphlogistique stéroïdien est la dexaméthasone

5. Procédé selon l'une quelconque des revendications précédentes, comprenant, en outre, l'incorporation, dans la composition, de 0,05 à 5 % en poids d'un agent antiphlogistique non-stéroïdien.

6. Procédé selon la revendication 5, dans lequel l'agent antiphlogistique non-stéroïdien est l'indométacine.

7. Procédé selon l'une quelconque des revendications précédentes, comprenant, en outre, l'incorporation, dans la composition, de 0,2 à 1 % en poids d'un agent antibiotique.

8. Procédé selon la revendication 7, dans lequel l'agent antibiotique est la néomycine, la bacitracine, le chloramphénicol ou la tétracycline.

9. Procédé selon l'une quelconque des revendications précédentes, comprenant, en outre, l'incorporation, dans la composition, de 0,05 à 15 % en poids d'au moins un épaississant choisi parmi l'hydroxypropylméthylcellulose, la méthylcellulose, la polyvinylpyrrolidone, l'alcool polyvinylique, les poly(alkylèneglycols), les poly[(méth)acrylates d'alkylèneglycol] et les poly(méth)acrylamides.

10. Procédé selon l'une quelconque des revendications précédentes, comprenant, en outre, la transformation de la composition en un médicament.

11. Procédé selon l'une quelconque des revendications précédentes, comprenant, en outre, l'imprégnation de la composition ou du médicament dans une matrice hydrophile ayant avantageusement la forme d'une bande ou d'une lentille de contact.

12. Utilisation d'une composition ou d'un médicament formé selon l une quelconque des revendications précédentes pour la fabrication d'un médicament pour un traitement anti-exsudatif, antiphlogistique et/ou antimicrobien en ophtalmologie, en otolaryngologie ou en dermatologie.
